(19) Europäisches Patentamt
European Patent Office
Office européen des brevets

(11) **EP 4 280 224 B1**

(12) **EUROPEAN PATENT SPECIFICATION**

(45) Date of publication and mention
of the grant of the patent:
**24.06.2026 Bulletin 2026/26**

(21) Application number: **22465534.0**

(22) Date of filing: **17.05.2022**

(51) International Patent Classification (IPC):
*G16H 10/20* (2018.01)    *G16H 30/40* (2018.01)
*G16H 40/63* (2018.01)    *G16H 50/20* (2018.01)
*G16H 50/30* (2018.01)    *G16H 50/70* (2018.01)

(52) Cooperative Patent Classification (CPC):
**G16H 50/30; G16H 10/20; G16H 30/40;**
**G16H 40/63; G16H 50/20; G16H 50/70**

(54) **A COMPUTER-IMPLEMENTED METHOD OF DETERMINING A MYOCARDIAL INFARCT RISK INFORMATION AND FOR PROVIDING A TRAINED RISK DETERMINATION FUNCTION, A RISK DETERMINATION SYSTEM AND COMPUTER PROGRAM**

COMPUTERIMPLEMENTIERTES VERFAHREN ZUR BESTIMMUNG VON MYOKARDINFARKT-RISIKOINFORMATIONEN UND ZUR BEREITSTELLUNG EINER TRAINIERTEN RISIKOBESTIMMUNGSFUNKTION, RISIKOBESTIMMUNGSSYSTEM UND COMPUTERPROGRAMM

PROCÉDÉ MIS EN OEUVRE PAR ORDINATEUR PERMETTANT DE DÉTERMINER UNE INFORMATION SUR LE RISQUE D'INFARCTUS DU MYOCARDE ET DE FOURNIR UNE FONCTION DE DÉTERMINATION DU RISQUE ENTRAÎNÉE, SYSTÈME DE DÉTERMINATION DE RISQUE ET PROGRAMME INFORMATIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**22.11.2023 Bulletin 2023/47**

(73) Proprietor: **Siemens Healthineers AG**
**91301 Forchheim (DE)**

(72) Inventors:
• **Berger, Martin**
**91088 Bubenreuth (DE)**
• **Hartung, Ulrich**
**91094 Langensendelbach (DE)**
• **Itu, Lucian Mihai**
**500294 Brasov (RO)**
• **Neumann, Dominik**
**91052 Erlangen (DE)**
• **Sharma, Puneet**
**Princeton Junction, 08550 (US)**

(74) Representative: **Siemens Healthineers**
**Patent Attorneys**
**Postfach 22 16 34**
**80506 München (DE)**

(56) References cited:
**EP-A1- 3 404 667      CN-A- 111 260 209**
**US-A1- 2015 112 182    US-A1- 2018 310 888**
**US-A1- 2021 251 577**

**Description**

A myocardial infarct (MI), commonly known as a heart attack, occurs when blood flow decreases or stops to the coronary artery of the heart, causing damage to the heart muscle. In 2015 worldwide, about 16 million myocardial infarctions occurred. An MI is one of the most expensive conditions during inpatient hospitalizations in the US. An MI may cause heart failure, an irregular heartbeat, cardiogenic shock or cardiac arrest.

Most MIs occur due to a coronary artery disease. There are countless risk factors e.g., high blood pressure, smoking, diabetes, lack of exercise, obesity, high blood cholesterol, poor diet and excessive alcohol intake. The risk of MI or other cardiac events is highly subject-specific. There exist scoring tools designed to predict cardiovascular risk (MI, stroke, other major heart disease) for certain subgroups of patients, for example the "Reynolds Risk Score". However, they are typically only taking into account limited data such as basic patient characteristics, smoking status, and laboratory data (from blood samples).

There are a number of tests to diagnose an MI, for example electrocardiograms (ECGs) and blood tests. An ECG, which is a recording of the heart's electrical activity, may confirm an ST elevation MI. Blood tests are usually based on troponin and less often on creatine kinase MB.

A coronary angiogram allows visualization of narrowings or obstructions on the heart vessels, and therapeutic measures can follow immediately. A chest radiograph and routine blood tests may indicate complications or precipitating causes and are often performed upon arrival to an emergency department. New regional wall motion abnormalities on an echocardiogram are also suggestive of a myocardial infarction. In stable patients whose symptoms have resolved by the time of evaluation isotopes like Technetium-99m, Thallium-201 chloride or Rubidium-82 chloride can be used to visualize areas of reduced blood flow.

MIs are often based on a coronary artery disease (CAD). A CAD is one type of cardiovascular disease, in which the coronary arteries, i.e., the arteries supplying the myocardium (heart muscle) with blood, are narrowed (stenosis), occluded, or otherwise impaired. Typically, obstructive stenoses (narrowing >50% in stenotic region compared to healthy reference diameter) are a strong indication for treatment, either by medication or intervention (stenting or bypass grafting). However, patients with stenoses considered non-obstructive or non-relevant (i.e., patients that are normally not selected for intervention), may still suffer from cardiac events such as myocardial infarction (MI) later in their life, and some of them could have benefitted from medication for risk reduction.

Detection and diagnosis of CADs is normally based on an invasive diagnostic coronary angiography. A coronary angiogram allows visualization of narrowings or obstructions on the heart vessels, and therapeutic measures can follow immediately. Angioplasty requires extensive skill, especially in emergency settings. It is performed by a physician trained in interventional cardiology.

Patients with negative coronary angiography, i.e., patients whose invasive coronary angiography does not indicate relevant (obstructive) stenoses, are often left in follow-up or treated not according to the guideline recommendation, even though they might benefit from medical therapy. However, it is not clear to what extent the existence, severity, quantity, and type of non-obstructive stenoses and other coronary artery abnormalities, such as wall irregularities, tortuosity, myocardial bridging, etc., affects the risk of cardiac events in the weeks, months and years following the exam.

To determine a personalized risk of an MI, findings from imaging and non-imaging data can be used. The precision of the determined risk depends on the used data. Accordingly, there is a need of advanced methods for determining a myocardial infarct risk. This need is met by the present invention, which allows combining multimodal data, including imaging and non-imaging data.

The document EP3404667A1 describes a computer-implemented method for providing a personalized evaluation of assessment of atherosclerotic plaques for a patient acquiring patient data comprising non-invasive patient data, medical images of the patient, and blood biomarkers. Features of interest are extracted from the patient data and one or more machine learning models are applied to the features of interest to predict one or more measures of interest related to atherosclerotic plaque.

In US2021251577A1 a machine-based risk prediction or assistance is described for peri-procedural complication, such as peri-procedural myocardial infarction (PMI). A machine-learned model is used to predict risk of PMI and/or recommend courses of action to avoid PMI in PCI. Various combinations of types or modes of information are used in the prediction, such as both imaging and non-imaging data. The prediction may be made prior to, during, and/or after PCI using the machine-learned model to more quickly reduce the chance of PMI. The workflows for prior, during, and/or post PCI incorporate the risk prediction and/or risk-based recommendations to reduce PMI for patients.

The document US2018310888A1 discusses the use of machine learning (ML) algorithms in predicting hemodynamic measures of interest in patients with acute coronary syndrome (ACS).

It outlines a method involving the extraction of patient-specific coronary geometry data and ACS-related features from medical images and additional patient measurement data, followed by the application of ML algorithms to predict patient-specific hemodynamic measures such as Fractional Flow Reserve (FFR), Coronary Flow Reserve, and risk of plaque rupture. It emphasizes the validation process through comparison with multiple imaging modalities.

The invention concerns a computer-implemented method of determining a myocardial infarct risk information and for providing a trained risk determination function, a risk determination system, a computer program and a medium with the computer program. Advantageous developments are given in the dependent claims and in the description that follows.

The invention will be described below both with regard to the claimed method and also with regard to a system (apparatuses). Features, advantages or alternate forms of embodiment mentioned here are likewise to be transferred to the other claimed subject matter and vice versa. In other words, the physical claims (which are directed to an system for example) can also be developed with the features that are described or claimed in conjunction with the method. The corresponding functional features of the method are embodied in such cases by corresponding physical modules.

The invention relates to a computer-implemented method for determining a myocardial infarct risk information for a patient based on medical imaging data and on supplementary data. Determining the myocardial infarct risk information is especially understood as providing and/or generating the myocardial infarct risk information.

The method comprises the method steps:

- obtaining and/or receiving medical imaging data comprising image data of at least a section of the heart of the patient,
- obtaining and/or receiving supplementary data comprising clinical, demographic, personal and/or survey data of the patient,
- applying a trained image analysis function to the medical imaging data, wherein image based raw risk features are generated,
- applying a trained data analysis function to the supplementary data, wherein supplementary raw risk features are generated,
- applying a trained risk determination function to the image based raw risk features and to the supplementary raw risk features, wherein the myocardial infarct risk information is generated,
- providing the determined myocardial infarct risk information.

The method comprises a method step of obtaining medical imaging data. Obtaining the medical imaging data may be configured as comprising a receiving of the medical imaging data. The medical imaging data may also be obtained by processing at least one medical image. The medical imaging data comprise image data and/or at least one image of a patient's heart. The image data and/or the image may comprise the whole heart or a section of the heart, e.g. coronary arteries. The medical imaging data and/or the image can be based on a medical imaging of a patient.

The method further comprises a method step of obtaining supplementary data. Obtaining the supplementary data may be configured s receiving of the supplementary data. The supplementary data may also be obtained by processing patient data or patient information. The supplementary data may comprise or are based on clinical, demographic, personal and/or survey data.

The medical imaging data and the supplementary data are obtained for the same patient, especially for the patient, whose myocardial risk should be determined.

The method comprises as a method step applying a trained image analysis function to the medical imaging data. This means especially, the medical imaging data are input data for the trained image analysis function. The image analysis function, especially the trained image analysis function, is configured as or is based on an algorithm, machine learning algorithm, deep learning algorithm and/or neural network. Preferably, the trained image analysis function is implemented or configured as a neural network, especially as a convolutional neural network. The trained image analysis function can be provided by a method for training such a function, by an app store or a provider for trained functions. The trained image analysis function can be further trained during usage, e.g. based on the medical imaging data. The trained image analysis function is configured to generate image based raw risk features. In other words, applying the trained image analysis function to the medical imaging data generates image based raw risk features. The image based raw risk features are especially raw risk features which are generated based on an image, especially based on the medical imaging data. The image based raw risk features depend on characteristics in the medical imaging data, especially the medical image, wherein the characteristics belong the attributes in an image which are based on or indicators for a myocardial infarct, stenosis or cardiovascular disease. The image based raw risk features may be configured as abstract features, e.g. they are not intuitively interpretable by a human. The image based raw risk features can be configured as a feature vector. The image based raw risk features can be configured as input data for a further trained function, machine learning algorithm, neural network, or deep learning method. The image based raw risk features are especially input data for the trained risk determination function.

The method comprise as a method step applying a trained data analysis function to the supplementary data. In other words, the supplementary data are input data for the trained data analysis function. The data analysis function, especially the trained data analysis function, can be configured as or be based on an algorithm, a machine learning algorithm, neuronal network and/or deep learning algorithm. The trained data analysis function is configured to generate supplementary raw risk features. The trained data analysis function is configured to analyze the supplementary data, especially to analyze the supplementary data for characteristics and/or attributes indicating or determining the myocardial risk of a patient. The supplementary raw risk features may be configured as a feature vector. The supplementary raw risk features depend on the supplementary data. The supplementary raw risk features can be configured as input data for a further trained function, machine learning algorithm, neural network, or deep learning method. The supplementary raw risk features are especially input data for the trained risk determination function.

In other words, the trained image analysis function is a trained function for image analysis and/or configured to analyze, e.g., to determine, features in or based on an image, especially medical image. In contrast, the trained data analysis function is especially for analyzing non-image data, for example for alphanumeric, text-based data or mixed data. Each of the two trained function is generating raw risk features, such that raw risk features based on image analyzes and based on non-image data analyzes are generated. In other words, raw risk features depending on attributes indicating a myocardial infarct or determining the myocardial infarct risk of patient are generated based on analyzing image and non-image data. These raw risk features are input to the trained risk determination function to generate a more practical and/or intuitively interpretable myocardial infarct risk information.

The method according to the invention comprises as a method step applying the trained risk determination function to the image based raw risk features and the supplementary raw risk features. In other words, the image based raw risk features and the supplementary raw risk features are input data for the trained risk determination function. The trained risk determination function generates myocardial infarct risk information. The myocardial infarct risk information describes, rates, numbers and/or estimates the personal risk of a patient to suffer a myocardial infarct in the future. The myocardial infarct risk information is determined based on image data, especially the medical imaging data, and on non-image data, especially the supplementary data.

In particular, a trainable function and/or a trained function emulates cognitive functions, which connect humans with human thought. In particular, training based on training data enables the trainable function to be adjusted to new circumstances and to recognize and extrapolate patterns.

Parameters of a trainable or trained function can be adjusted by means of training. In particular, supervised training, semi-supervised training, unsupervised training, reinforcement learning and/or active learning can be used for this. In particular, the parameters of the trainable functions can be adjusted iteratively by a number of training steps.

A trainable or trained function can in particular comprise a neural network, a support vector machine, a random tree or a decision tree and/or a Bayesian network, and/or the trainable function can be based on k-means clustering, Q-learning, genetic algorithms and/or association rules. A trainable or trained function can in particular comprise a combination of a number of uncorrelated decision trees or an ensemble of decision trees (random forest). In particular the trainable or trained function can be determined by means of XGBoosting (extreme Gradient Boosting). In particular a neural network can be a deep neural network, a convolutional neural network or a convolutional deep neural network. What is more a neural network can be an adversarial network, a deep adversarial network and/or a generative adversarial network. In particular a neural network can be a recurrent neural network. In particular a recurrent neural network can be a network with long short-term-memory, LSTM, in particular a Gated Recurrent Unit (GRU). In particular a trainable or trained function can comprise a combination of the approaches described.

The medical imaging data in particular comprise a presentation of an examination object. In particular, the medical imaging data and the supplementary data represent the same examination object and/or patient. The examination object in this case can in particular be a person, for example a patient, an animal or an object. In particular, the examination object can be a part of the patient, the person or the animal or the object. For example, the examination object can be a heart or a thorax of the examination object. The medical imaging data can in particular comprise two-dimensional (2D), three-dimensional (3D) and/or four-dimensional (4D) medical image data.

2D medical image data can in particular be pixelated image data. In particular 2D medical image data can comprise a pixel matrix, wherein each pixel of the pixel matrix is assigned at least one image value. 2D medical image data can for example comprise a projection image or a slice image from the X-ray imaging. For X-ray imaging the medical modality corresponds to the X-ray system. In the imaging protocol in this case for example an X-ray voltage, a current strength, an illumination time etc. can be predetermined. As an alternative 2D medical image data can for example comprise image data from an ultrasound examination or a sonography. The medical modality in this case corresponds to the ultrasound system. The imaging protocol can for example in this case predetermine an ultrasound head used, a frequency and/or a type of data acquisition etc. The type of data acquisition specifies for example whether Doppler ultrasound is involved, and/or whether the data is acquired in an A mode or a B mode or a M mode. As an alternative 2D medical image data can for example comprise tissue slices from histopathology. In particular the tissue slices can be stained or unstained. The medical modality used in this case corresponds to an optical microscope.

3D medical image data can in particular be voxelated image data. In particular 3D medical image data can comprise a three-dimensional voxel matrix, wherein each voxel of the voxel matrix is assigned at least one image value. 3D medical image data can in particular comprise image data from a computed tomography examination and/or a magnetic resonance tomography examination and/or a positron emission tomography examination and/or a single photon emission computed tomography examination and/or a 3D ultrasound examination or 3D sonography. In other words, 3D medical image data represents the examination object, especially the heart, in three spatial dimensions. The different imaging protocols can in particular predetermine parameters for recording or for acquisition of the medical image data with the respective medical modality.

4D medical image data can in particular comprise 3D medical image data with an additional time component. Furthermore, 4D medical image data can be understood in a broad interpretation as 2D medical image data with time component. In other words, 4D medical image data typically comprises time-resolved 3D or 2D medical image data or a chronological series of 3D or 2D medical image data. In particular 4D medical data comprises a time vector which comprises at least one point in time. In particular each point in time comprised by the time vector can be assigned a 3D medical image dataset.

The supplementary data can in particular comprise an electrocardiogram (acronym: EKG), which can be acquired with an electrocardiography system. In an EKG at least one value is assigned within a period of time to one or more discrete points in time. In an EKG the image value can correspond to a potential acquired by the electrocardiography system at a corresponding point in time. Heart activity in particular can be represented in this way.

In the method step of providing the determined myocardial infarct risk information, the myocardial infarct risk information determined for the patient and/or determined based on the medical imaging data and the supplementary data is provided. In particular the myocardial infarct risk information is provided as data for storage or transfer, as report, value or image displayed on a monitor. In particular the myocardial infarct risk information can be provided for a use by means of any given computing unit.

The inventor has recognized that it is possible and advantageous to use image data and non-image data for determining a myocardial infarct risk information. In particular the inventor has recognized to use three trained functions for robust determining a significant myocardial infarct risk information.

According to the invention the method step of applying the trained image analysis function to the medical imaging data generates the image based raw risk features and at least one first confidence value. Preferably, applying the trained image analysis function to the medical imaging data generates for each image based raw risk feature at least one confidence value. The first confidence value is a measure for the confidence and/or reliability of the generated image based raw risk features. For example, a generated image based raw risk feature which is based on weak attribute in the image has a small confidence value.

The method step of applying the trained data analysis function to the supplementary data generates the supplementary raw risk features and at least one second confidence value. Especially, the trained data analysis function is configured to generate for each supplementary raw risk feature at least one confidence value. The first confidence value is a measure for the confidence and/or reliability of the generated image based raw risk features. The first and/or the second confidence value is preferably a real number, especially a percentage.

The trained risk determination function is applied to the image based raw risk features, the at least one first confidence value, the supplementary raw risk features and the at least one second confidence value to generate the myocardial infarct risk value. In particular, applying the trained risk determination function generates the myocardial infarct risk information and at least one third confidence value, wherein the third confidence value is a measure for the confidence and/or reliability of the generated myocardial infarct risk information. The myocardial infarct risk information is particularly provided together with the third confidence value.

This is based on the consideration, to determine for the image analysis function and the data analysis function separately at least one confidence value, which are input to the risk determination function. The trained risk determination function can for example use the confidence values to weight the influence of features on the myocardial infarct risk information.

In particular, the myocardial infarct risk information comprises, describes and/or is a probability, especially it is a percentage value or a value between 0 and 1. Especially, the third confidence value comprises, describes and/or is a reliability of the probability comprised and/or described by the myocardial infarct risk information. The probability of the myocardial infarct risk information and/or the myocardial infarct risk information is a probability for the patient, especially for an occurrence of a myocardial infarct, of hospitalization due to a myocardial infarct and/or death due to a myocardial infarct. The probability, especially for the occurrence, hospitalization and/or death can be generated for fixed time interval in the future (e.g. 2 years) or for an adjustable time interval in the future (e.g. days, weeks, month, years).

Especially, the method is configured to generate myocardial infarct risk information comprising or concerning different time intervals, e.g. one for half a year in the future and one for 5 years in the future. Preferably, the method and/or the trained risk determination function is configured to generate myocardial infarct risk information comprising or concerning different time intervals, e.g. one for half a year in the future and one for 5 years in the future.

According to a preferred embodiment of the invention, the medical imaging data comprise coronary angiography data. Especially, the medical imaging data comprise a coronary angiogram. The trained image analysis function is herein applied to the coronary angiography data. The trained image analysis function is for example configured to detect, segmentate and/or label the coronary angiography data, especially the coronary angiogram. In particular, the trained image analysis function is configured to determine, segmentate, classify, localize, and/or label in or based on the medical imaging data, especially the coronary angiography data, a stenosis, a level of an artillery narrowing, an artery abnormality, wall irregularities, tortuosity and/or myocardial bridging. A stenosis is a narrowing of an artery, especially of a coronal artery. The level of narrowing is for example the percentage of the narrowing, especially compared to the unnarrowed artery. Wall irregularities are for example irregularities of the heart wall, for example necrotic tissue or tissue insufficient supplied with blood.

According to a preferred embodiment of the invention, the supplementary data, especially the clinical, demographic, personal and/or survey data of the patient, comprise blood values, especially blood values taken on different dates. Furthermore, the supplementary data, especially the clinical, demographic, personal and/or survey data of the patient, may comprise genetic information of the patient, for example a genetic sequencing or findings of genetic markers. Furthermore, the supplementary data, especially the clinical, demographic, personal and/or survey data of the patient, may comprise information or data about the medical condition of the patient, for example, weight, blood pressure, height, nutrition, medication and/or regular sports activities. Furthermore, the supplementary data, especially the clinical, demographic, personal and/or survey data of the patient, may comprise information about the patient's smoking status, e.g., a general smoking status, smoker or non-smoker, or a more detailed information like number of cigarettes per day. The supplementary data, especially the clinical, demographic, personal and/or survey data of the patient, may comprise also information about potential risk factors for a myocardial infarct, like family history and/or living style of the patient.

The supplementary data, especially the clinical, demographic, personal and/or survey data of the patient, may comprise processed and/or unprocessed data. The supplementary data, especially the clinical, demographic, personal and/or survey data of the patient, comprise preferably information of scoring result or the scoring result. The scoring result is for example based on scoring tools designed to predict a cardiovascular risk (e.g., MI, stroke, other major heart disease). The scoring result is for example based on the known "Reynolds Risk Score". The scoring result can be based on a SYNTAX score, which is for example known from Suh, Young Joo et al. "SYNTAX score based on coronary computed tomography angiography may have a prognostic value in patients with complex coronary artery disease: An observational study from a retrospective cohort." (Medicine vol. 96,37 (2017): e7999.

doi:10.1097/MD.0000000000007999) .

In particular, the patient can be equipped with a wearable device, wherein the wearable device can be a smart device, smart phone, fitness tracker, medical tracker and/or sensor. The wearable device is configured to provide update data. The update data comprise, for example, an actualization of at least a subsection of the supplementary data. The update data are provided at a later time than and/or after applying the trained risk determination function to the image based raw risk features and to the supplementary raw risk features. For example, the update data are provided and/or received at least 1 month or 1 year after determining the myocardial risk information based on the medical imaging data and the supplementary data. Preferably, the update data are provided and/or received periodically, e.g., every month. The update data can comprise vital parameters of the patient, blood pressure, tracked activities, tracked sports activities, sleeping information and/or information about taken medication. The wearable device may also be configured to support or apply medical treatment, for example for depot medication or insulin pump, wherein the wearable device provides as update data information about the applied medical treatment, concentration of medication or measured data. Furthermore, the update data may be provided by a user filled questionary, wherein the user or patient answers the questionary on the wearable device, a computer, tablet and/or smart phone.

The update data can be used to determine and/or generate the myocardial infarct risk information and/or to generate the supplementary raw features, especially to generate the second and/or third confidence value. Preferably, the update data are used to update the generation of the myocardial infarct risk information. In particular, the update data are used to update the supplementary data, e.g., by replacement of old data or by cumulating old data with the update data. Optionally, the trained risk determination function is configured to determine an updated myocardial infarct risk information as the myocardial infarct risk information when applied to the image based raw risk features, the supplementary raw risk features and the update data. Particularly, the updated myocardial infarct risk information is compared to the determined myocardial infarct risk information, especially a previously determined updated myocardial infract risk information, and/or an threshold value, wherein for example an alarm is given, when the updated myocardial infarct risk information exceeds the threshold, determined myocardial infarct risk information and/or the previously determined updated myocardial infract risk information.

The trained image analysis function can be configured for an automatic assessment of coronary angiography exams. Especially, trained image analysis function can comprise an AI-based image processing module, wherein module can herein be understood as algorithm or section of the trained function. For example, the image processing module and/or the trained image analysis function

- is based on or comprises a view classification, a vessel segmentation, a branch labeling, a stenosis detection, a temporal aggregation and/or computes a temporally consistent list of stenosis findings, including stenosis grade, location.
- is configured to compute the topology of the full coronary artery tree, which can help in localizing abnormalities in a structured manner.
- is configured to compute vessel wall segmentation for individual segments, branches, or the full coronary tree, which can be used to identify abnormal patterns (wall irregularities, tortuosity, other abnormalities, even novel types of abnormalities discovered by the AI), or more generically, to characterize the vascular tree.
- is configured for automatic functional assessment, e.g., to derive image-based FFR (fractional flow reserve).
- is configured to assess the quality of an exam, especially image quality and/or foreshortening, to control the relative contribution of a specific sequence to the risk feature extraction training. For example, low quality -> reduced confidence in results of fully automatic pipeline -> reduce influence of these results on the trained model, e.g., by assigning a low relative weight to this data during training.

In particular, the trained risk determination function can be configured to indicate possible treatments for the generated myocardial infarct risk information. For example:

- Low risk: lifestyle changes
- Medium risk: lifestyle changes + statins
- High risk: lifestyle changes + statins + colchicine

Naturally, the risk of future events is directly influenced by the adherence to lifestyle change recommendations and by the compliance to the prescribed medical treatment. Hence, once the patient is discharged from the hospital, a continuous monitoring system may be employed which updates the risk, based on e.g.:

- Wearable devices: monitoring physical activity, fitness, etc. (monitoring adherence to lifestyle changes)
- App providing periodic questionnaires to the patient, which collect information on medical treatment compliance.

In an advantageous embodiment, a separate risk is computed for each type of event: death, MI, hospital readmission, etc. A key element in the differentiation of the different risks is represented, e.g., by the collateralization capabilities of the coronary circulation. Collaterals are inter-arterial connections that provide blood flow to a myocardial territory with an obstruction in the original supply vessel.

Clinical studies have demonstrated that a well-functioning coronary collateral circulation independently predicts lowered mortality in patients with chronic CAD and acute CAD. The protective effect translates into improved left ventricular (LV) function (e.g., higher EF), decreased remodeling, and a lower risk of arrhythmias.

The herein proposed method may also be employed for patients who have undergone an intervention (PCI, CABG) to predict their risk of future events, e.g., given all patient characteristics and characteristics of mild/minimal stenoses, which have been left untreated.

The invention moreover relates to a computer-implemented method for providing a trained risk determination function, especially providing it for the computer-implemented method to determine myocardial infarct risk features. The computer-implemented method, for shortening also just called method, comprises the method steps:

- receiving image based raw risk features derived from medical imaging data of at least a section of the heart of a patient,
- receiving supplementary raw risk features derived from supplementary data comprising clinical, demographic, personal and/or survey data of the patient,
- receiving input myocardial infarct risk information of the patient,
- applying a trained risk determination function to the image based raw risk features and to the supplementary raw risk features, wherein output myocardial infarct risk information is generated,
- training the trained risk determination function based on a comparison of the input myocardial infarct risk information and the output myocardial infarct risk information,
- providing the trained risk determination function.

The image based raw risk features can be provide s data set, e.g., by a storage medium or sample set. The image based raw risk features can also be provided as processing result, e.g., result of applying an image analysis function to image data. Generation of the provided and received image based raw risk features can be part of the method. The image based raw risk features are derived from medical imaging data of at least a section of the heart of a patient. The received image based raw risk features comprise at least one first confidence value. The received image based raw risk features are based on and/or configured as the generated image based raw risk features described for the computer-implemented method for determining the myocardial infarct risk information. The received image based raw risk features are derived from image data.

The supplementary raw risk features can be provided as data set, e.g., by a storage medium or sample set. The supplementary raw risk features can also be provided as processing result, e.g., result of applying a data analysis function to supplementary and/or non-image data. Generation of the provided and received supplementary raw risk features can be part of the method. The supplementary raw risk features are derived from supplementary data, especially from clinical, demographic, personal and/or survey data. The received supplementary raw risk features comprise at least one second confidence value. The received supplementary raw risk features are based on and/or configured as the generated supplementary raw risk features described for the computer-implemented method for determining the myocardial infarct risk information. The received image based raw risk features are derived from non-image data.

According to a method step, input myocardial infarct risk information of the patient is received. The input myocardial infarct risk information is related to the supplementary and image based raw risk features. Preferably, the input myocardial infarct risk information describes the risk and/or probability of a person to have a myocardial infarct, wherein this person would provide and/or have image based raw risk features as received and supplementary raw risk features as received.

To generate output myocardial infarct risk features, a trained, especially pre-trained, risk determination function is applied to the image based raw risk features and to the supplementary raw risk features. According to one of the method steps, training of the trained risk determination function is based on a comparison of the input myocardial infarct risk information and the output myocardial infarct risk information. The trained risk determination function is provided, e.g., provided for usage in the computer-implemented method for determining the myocardial infarct risk information based for a patient.

The comparison of the myocardial infarct risk information and the output myocardial infarct risk information can for example compare the deviation of the risk information and/or the probabilities. The comparison is preferably configured as a measure of deviation or similarity, e.g., based on a deviation or similarity function. The output myocardial infarct risk information comprises for an example a probability $P_1$, estimating the probability for a patient with the image based raw risk features and the supplementary raw risk features to suffer a myocardial infarct, wherein the myocardial infarct risk information comprises the real, known, verified or more realistic probability $P_2$ of the same patient to suffer a myocardial infarct, wherein the comparison is for example the deviation $\Delta = P_1 - P_2$. The output myocardial risk information, especially the value of probability $P_1$, depend in particular on the at least one parameter $\omega$ of the trained risk determination function. In other words, a change in the at least one parameter leads to a change in output myocardial infarct risk information the values of the synthetic image data. The measure of similarity $\Delta(\omega)$ can for example be calculated as follows:

$$\Delta(\omega) = P_1(\omega) - P_2$$

In particular it is true to say that the greater is the measure of similarity, the better is the output myocardial infarct risk information. In particular the measure of similarity depends on the at least one parameter of the trainable function.

In the method step of training the trained risk determination function at least one parameter of the trained risk determination function is preferably adjusted. The at least one parameter of the trained risk determination function is adjusted based on the comparison of the output myocardial infarct risk information and the myocardial infarct risk information, especially based on the measure of similarity and/or based on the deviation $\Delta$ or $\Delta(\omega)$. In particular the at least one parameter is adjusted in such a way that the deviation $\Delta$ or $\Delta(\omega)$ is decreased, in particular minimized. In particular the output myocardial infarct risk information, in particular the probability $P_1$, can be varied or adjusted through adjustment of the at least one parameter in such a way that the deviation between the myocardial infarct risk information and the output myocardial infarct risk information determined by application of the adjusted trained risk determination function is reduced, in particular minimized.

In particular the method steps of applying a trained risk determination function, generating the output myocardial infarct risk information, comparing the output myocardial infarct risk information and adjusting the at least one parameter of the trained risk determination function is carried out iteratively. In each iteration step in particular the trained risk determination function is optimized by adjustment of the at least one parameter. In the subsequent iteration the output myocardial infarct risk information is determined by application of the optimized trained risk determination function. Subsequently the comparison and/or the deviation between the output myocardial infarct risk information and the myocardial infarct risk information is again determined. Based on this comparison and/or deviation the at least one parameter of the trained function is adjusted once again. In this case the trained risk determination function is again optimized in such a way that the output myocardial infarct risk information more strongly resembles myocardial infarct risk information.

In the method step of providing the trained risk determination function, the function able to be trained in the way described above is provided. In particular the trained function is provided for determining myocardial infarct risk information. In particular the trained risk determination function can be provided for a use. In particular the trained risk determination function can be provided for a use by means of any given computing unit.

The inventor has recognized that it is possible to train the risk determination function and/or to adjust the at least one parameter of the trained risk determination function based on the comparison of the myocardial infarct risk information and the output myocardial infarct risk information.

In an optional embodiment of the invention, the method comprises as method step a receiving of image training data. The image training data are preferably structured as the medical imaging data of the method for determining the myocardial infarct risk information. The image training data are for example provided from a data storage and/or from a training data sample set. The image training data comprise medical image data of at least a section of a heart. Preferably, the image training data comprise coronary angiography data and/or a coronary angiogram.

Furthermore, the method can comprise a method step of receiving supplementary training data, wherein the supplementary training data comprise clinical, demographic, personal and/or survey data. The supplementary training data are preferably structured as the supplementary data of the method for determining the myocardial infarct risk information. The supplementary training data are for example provided from a data storage and/or from a training data sample set.

In particular, the method comprises as a method step, receiving input image based raw risk features and/or input supplementary raw risk features related to the training image data and/or to the supplementary training data are. Preferably, the input image based raw risk features describe or are based on features in the training image data relating or indicating a myocardial infarct. In particular, the input supplementary raw risk features describe or are based on features in the supplementary training data relating or indicating a myocardial infarct.

To generate output image based raw risk features a trained, especially pre-trained, image analysis function is applied to the training image data, wherein the output image based raw risk features are generated, wherein preferably also at least one output first confidence value is generated. To generate output supplementary raw risk features a trained, especially pre-trained, data analysis function is applied to the supplementary training data, wherein the output supplementary raw risk features are generated, wherein preferably also at least one output second confidence value is generated.

According to a preferred embodiment, the method comprises as a method step, training of the trained image analysis function based on a comparison of the output image based raw risk features and the input image based raw risk features. The method comprises as a method step preferably, training of the trained data analysis function based on a comparison of the output supplementary raw risk features and the input supplementary raw risk features. The training comprises for example an adjustment of at least one parameter of the trained image analysis function and/or of the trained data analysis function. The training and/or the adjustment of the parameter is especially based and/or configured as described for the training of the risk determination function. The trained image analysis function and/or trained data analysis function is provided, e.g., provided for usage in the computer-implemented method for determining the myocardial infarct risk information based for a patient.

In particular, the output image based raw risk features generated by applying the trained image analysis function to the training image data are provided as the image based raw risk features for the training of the trained risk determination function. In other words, the output image based raw risk features are received as the image based raw risk features in the method step of receiving the image based raw risk features. Preferably, the output supplementary raw risk features generated by applying the trained data analysis function to the supplementary training data are provided as the supplementary raw risk features for the training of the trained risk determination function. In other words, the output supplementary raw risk features are received as the supplementary raw risk features in the method step of receiving the supplementary raw risk features. The training of the trained risk determination function is in particular based on the training image data and the supplementary training data. Furthermore, the trained risk determination function can in particular be trained together with the trained image analysis function and/or the trained data analysis function. Alternatively, the trained risk determination function, the trained image analysis function and/or the trained data analysis function are trained separately.

The input myocardial infarct risk information for the training of the risk determination function, the input image based raw risk features and/or the input supplementary raw risk features are especially based on or determined based on mortality data at least one former patient, data of hospitalization of at least one former patient and/or interviews or surveys with former patients or relatives of former patient. Even if for a subset of patients, the endpoint (myocardial infarct risk information) is unknown or uncertain, the clinical and/or imaging data collected at the time of the invasive diagnostic coronary angiography exam (or during other patient encounters) can still be leveraged to train the trained image analysis function and/or the trained data analysis function.

In a preferred embodiment of the invention, data programming may be employed for the programmatic creation and modeling of training datasets, especially of the input myocardial infarct risk information, the input image based raw risk features and/or the input supplementary raw risk feature. Thus, noisy training labels may be exploited by specifically encoding the weak supervision in the form of labeling functions. The data programming is preferably based on and/or configured as described by Alexander Ratner, Christopher De Sa, Sen Wu, Daniel Selsam, Christopher Ré in "Data Programming: Creating Large Training Sets, Quickly" (arxiv.org/abs/1605.07723). Labeling functions may have widely varying error rates and may conflict on certain data points. They can be modeled as a generative process, leading to an automated denoising by learning the accuracies of the labeling functions along with their correlation structure. A labeling function need not have perfect accuracy or recall; rather, it represents a pattern that the user wishes to impart to their model and that is easier to encode as a labeling function than as a set of hand-labeled examples. Importantly, labeling functions can overlap, conflict, and even have dependencies which users can provide as part of the data programming specification.

For example, labelling functions may be defined based on long-term patient outcome, but also based on plaque characteristics extracted from either CCTA or intravascular imaging (OCT, IVUS). Especially certain plaque characteristics are known to be of high risk (napkin ring sign, mixed plaque, positive remodeling, etc.).

The invention moreover relates to a risk determination system for determining a myocardial infarct risk information. The risk determination system comprises an interface and a computing unit. In this case the interface is embodied for receiving and/or obtaining medical imaging data and supplementary data. In this case the medical imaging data comprise an image or image data for a section of the patient's heart. The supplementary data comprise clinical, demographic, personal and/or survey data of the patient. In this case the computing unit is embodied to generate the myocardial infarct risk information, especially to execute and/or to apply the method for determining the myocardial infarct risk information according to the invention. The computing unit is configured to applying a trained image analysis function to the medical imaging data, wherein image based raw risk features are generated. The computing unit is configured to applying a trained data analysis function to the supplementary data, wherein supplementary raw risk features are generated. The computing unit is configured to applying a trained risk determination function to the image based raw risk features and to the supplementary raw risk features, wherein the myocardial infarct risk information is generated. The generated myocardial infarct risk information is provided, e.g., provided to a user and/or by the interface.

The invention also relates to a computer program product with a computer program as well as a computer-readable medium. A realization of the product largely in software has the advantage that even training systems previously used can be easily upgraded by a software update, in order to work in the way described. Such a computer program product, as well as the computer program, might possibly comprise additional elements such as e.g., documentation and/or additional components, as well as hardware components, such as e.g., hardware keys (dongles etc.) for using the software.

In particular the invention also relates to a computer program product with a computer program, which is able to be loaded directly into a memory of a risk determination system, with program sections for carrying out all steps of the method described above for determining a myocardial infarct risk information and its aspects when the program sections are executed by the risk determination system.

In particular the invention relates to a computer-readable memory medium, on which program sections able to be read and executed by a risk determination system are stored, for carrying out all steps of the method described above for determining a myocardial infarct risk information its aspects when the program sections are executed by the risk determination system.

The characteristics, features and advantages of this invention described above will become clearer and easier to understand in conjunction with the following figures and their descriptions. In this case the figures and descriptions are not intended to restrict the invention and its forms of embodiment in any way.

In different figures the same components are provided with corresponding reference characters. As a rule, the figures are not true-to-scale.

In the figures:

Figure 1 shows an exemplary embodiment of a method for determining a myocardial infarct risk information;

Figure 2 shows an exemplary first embodiment of a method for a training of a trained risk determination function;

Figure 3 shows an exemplary second embodiment of a method for a training of a trained risk determination function;

Figure 4 shows an exemplary risk determination system.

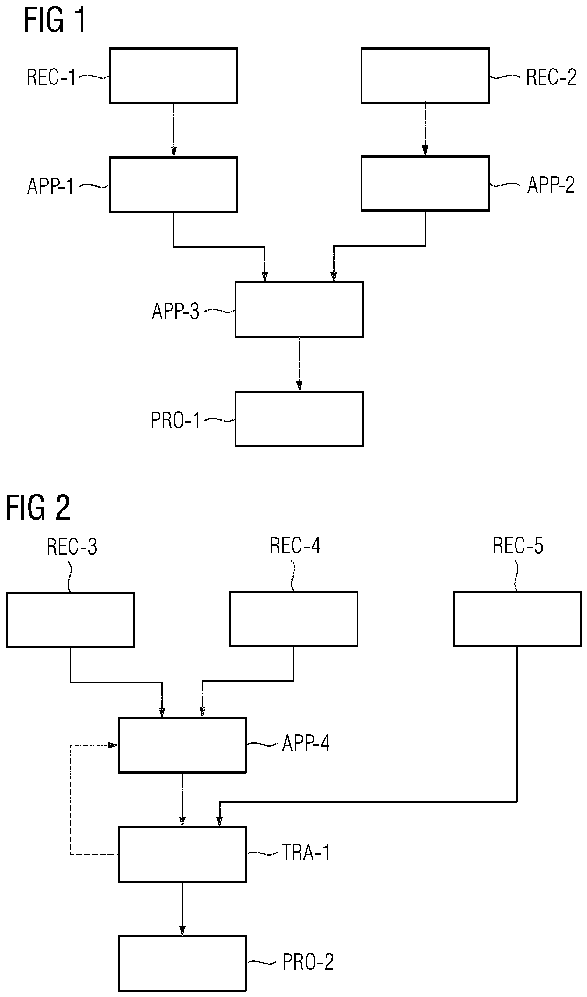

Figure 1 shows a first exemplary embodiment of a method for provision of myocardial infarct risk information for patient.

In a method step of receiving REC-1 medical imaging data is received by means of an interface SYS.IF.

In a method step of receiving REC-2 supplementary data is received, in particular by means of the interface SYS-IF.

In this case the method steps of receiving REC-1 the medical imaging data and of receiving REC-2 the second supplementary data can also be carried out at the same time or in the reverse order.

In this case the medical imaging data is based on medical imaging of an examination object of a patient. The examination object comprises in this case the heart of the patient. The medical imaging data comprise a coronary angiogram of the patients heart. In particular, the medical imaging data comprise magnetic resonance imaging data and/or computed tomography imaging data of the patients heart.

The supplementary data are non-image data, especially comprising only text and/or numbers. The supplementary data in particular don't comprise image data or images. In this case the supplementary data comprise clinical, demographic, personal and/or survey data of the patient.

The received medical imaging data and the received supplementary data are belonging to the same patient. The medical imaging data and/or the received supplementary data may comprise data and/or information aggregated over a time interval, e.g., over several months or years.

The received medical imaging data is used as input data for a trained image analysis function in the method step APP-1. The trained image analysis function is configured to detect features and/or image section indicating and/or prefiguring an increased risk of suffering a myocardial infarct. Applying the trained image analysis function in step APP-1 generates image based raw risk features and preferably at least one first confidence value. The generated image based raw risk feature and preferably the first confidence value is provided as input data to the method step APP-3.

The received supplementary data is used as input data for a trained data analysis function in the method step APP-2. The trained data analysis function is configured to detect features and/or data indicating and/or prefiguring an increased risk of suffering a myocardial infarct. Applying the trained data analysis function in step APP-2 generates supplementary raw risk features and preferably at least one second confidence value. The generated supplementary raw risk feature and preferably the second confidence value is provided as input data to the method step APP-3.

The generated and provided supplementary raw risk features and image based raw risk features, especially the first and second confidence value, is used as input data for a trained risk determination function in method step APP-3. Applying the trained risk determination function in step APP-3 generates, especially determines, a personalized myocardial infarct risk information based on the supplementary raw risk features and the image based raw risk features, preferably also based on the first and second confidence value. The myocardial infarct risk information describes and/or numbers a probability to suffer a myocardial infarct for the patient forming the basis of the medical imaging data and the supplementary data.

The generated and/or determined myocardial infarct risk information is provided in method step PRO-1, e.g., provided for usage, user interpretation and/or to determine a medical treatment. The myocardial infarct risk information is for example provided via the interface SYS-IF.

Figure 2 shows an exemplary first embodiment of a method for a training of a trained risk determination function, especially the trained risk determination function used in method step APP-3.

In a method step REC-3 image based raw risk features derived from medical imaging data of at least a section of the heart of a patient are received. The image based raw risk features are for example generated by applying a trained image analysis function to medical imaging data of a patient, wherein this patient is especially known person or patient with known myocardial infarct risk information.

In a method step REC-4 supplementary raw risk features derived from supplementary data of the patient belonging to the imaged based raw risk features are received. The supplementary raw risk features are for example generated by applying a trained data analysis function to supplementary data of the patient, wherein this patient is especially known person or patient with known myocardial infarct risk information.

In a method step REC-5 input myocardial infarct risk information are received. The input myocardial infarct risk information is the known or as correct assumed risk or probability to suffer a myocardial infarct for the patient belonging to the supplementary raw risk features and the image based raw risk features.

In a method step APP-4 a trained risk determination function is applied to the received image based raw risk features and the supplementary raw risk features, wherein output myocardial infarct risk information are generated and/or determined. Especially, also first and second confidence values for the supplementary raw risk features and for the image based raw risk feature are inputted to the risk determination function to generate and/or determine the myocardial infarct risk information.

In a method step TRA-1 the trained risk determination function is trained based on the output myocardial infarct risk information and the input myocardial infarct risk information. In this case, the input myocardial infarct risk information is compared with the output myocardial infarct risk information. For example, the output myocardial infarct risk information comprises a probability $P_O$ for suffering the myocardial infarct and the input myocardial infarct risk information comprises a probability $P_I$ for suffering the myocardial infarct, wherein for the comparison the deviation $\Delta = P_O - P_I$ is determined, wherein the trained risk determination function is trained such that the deviation is minimal $\Delta = P_O - P_I$.

The trained risk determination function of step TRA-1 is provided for use in method step PRO-2, especially provide for usage in method step APP-3 of figure 1.

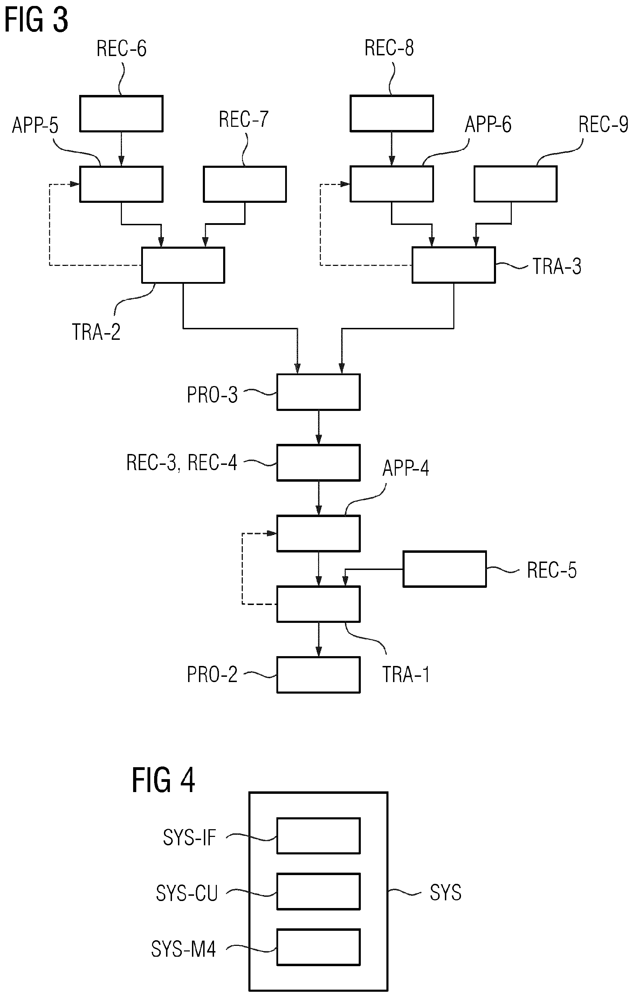

Figure 3 shows an exemplary second embodiment of a method for a training of a trained risk determination function, especially the trained risk determination function used in method step APP-3.

In a method step REC-6 medical imaging data of at least a section of the heart of a patient is received. The medical image data comprise a coronal angiogram of the patient.

In a method step APP-5 a trained image analysis function is applied to the medical image data received in step REC-6, wherein output image based raw risk features are generated and provided. The output image based raw risk features are provided to a training method step TRA-2.

In a method step REC-7 input image based raw risk features belonging the received medical imaging data of step REC-6 are received. The input image based raw risk features are the known or as correct assumed image based raw risk features for the medical imaging data.

In a method step TRA-2 a trained image analysis function is trained based on the output image based raw risk features and the output image based raw risk features, especially by comparing the input and output image based raw risk features. The trained image analysis function is preferably trained to minimize a deviation of the input and output image based raw risk features.

In a method step REC-8 supplementary data of a patient is received. The supplementary data comprise clinical, demographic, personal and/or survey data of the patient.

In a method step APP-6 a trained data analysis function is applied to the supplementary data received in step REC-8, wherein output supplementary raw risk features are generated and provided. The output supplementary raw risk features are provided to a training method step TRA-3.

In a method step REC-9 input supplementary raw risk features belonging the received supplementary data of step REC-8 are received. The input supplementary raw risk features are known or as correct assumed supplementary raw risk features for the received supplementary data.

In a method step TRA-3 a trained data analysis function is trained based on the output supplementary raw risk features and the output supplementary raw risk features, especially by comparing the input and output supplementary raw risk features. The trained data analysis function is preferably trained to minimize a deviation of the input and output supplementary raw risk features.

In a method step PRO-3 the output image based raw risk features are provided to the step REC-1 as the image based raw risk features for the training according to the method described for figure 2. In the method step PRO-3 the output supplementary raw risk features are provided to the step REC-1 as the supplementary raw risk features for the training according to the method described for figure 2. Alternatively, the input image based raw risk features and the input supplementary raw risk features are provided to the step REC-1 for the training of the risk determination function. Furthermore, the trained image analysis function and the trained data analysis function are provided in step PRO-3 for further use, especially for usage in Step APP-1 and APP-2 according to figure 1.

Figure 4 shows a risk determination system SYS for determining a myocardial infarct risk information.

The risk determination system SYS is embodied to carry out an inventive method for determining the myocardial infarct risk information for a patient. The risk determination system SYS comprises an interface SYS-IF, a computing unit SYS-CU and a memory unit SYS-MU.

The risk determination system SYS can in particular be a computer, a microcontroller or integrated circuit (IC). As an alternative the risk determination system SYS can be a real or virtual computer network (a technical term for a real computer network is a cluster, a technical term for a virtual computer network is cloud). The risk determination system SYS can be embodied as a virtual system, which can be embodied on a computer or a real computer network or a virtual computer network (a technical term is virtualization).

The interface SYS-IF can be a hardware or software interface (for example a PCI bus, USB or Firewire). The computing unit SYS-CU can comprise hardware and/or software elements, for example a microprocessor or what is known as an FPGA (Field Programmable Gate Array). The memory unit SYS-MU can be embodied as Random Access Memory (RAM) or as permanent mass storage (hard disk, USB stick, SD card, Solid State Disk (SSD)).

The interface SYS-IF can in particular comprise a plurality of sub-interfaces, which carry out various method steps of the respective inventive method. In other words, the interface SYS-IF can be embodied as a plurality of interfaces SYS-IF. The computing unit SYS-CU can in particular comprise a number of sub-computing units, which carry out various method steps of the respective inventive method. In other words, the computing unit SYS-CU can be embodied as a plurality of computing units SYS-CU.

Where this has not happened explicitly but is sensible and in the spirit of the invention, individual exemplary embodiments, individual or their part aspects or features can be combined with one another or interchanged without departing from the framework of the current invention. Advantages of the invention described with regard to one exemplary embodiment, also apply, where they can be transferred, without this being explicitly stated, to other exemplary embodiments.

**Claims**

1. A computer-implemented method of determining a myocardial infarct risk information for a patient based on medical imaging data and on supplementary data, the method comprising:

   - receiving (REC-1) medical imaging data of at least a section of the heart of the patient,
   - receiving (REC-2) supplementary data comprising clinical, demographic, personal and/or survey data of the patient,
   - applying (APP-1) a trained image analysis function to the medical imaging data, wherein image based raw risk features are generated, wherein applying (APP-1) the trained image analysis function to the medical imaging data generates a first confidence value for the generated image based raw risk features,
- applying (APP-2) a trained data analysis function to the supplementary data, wherein supplementary raw risk features are generated, wherein applying (APP-2) the trained data analysis function to the supplementary data generates a second confidence value for the generated supplementary raw risk features,
- applying (APP-3) a trained risk determination function to the image based raw risk features and to the supplementary raw risk features, wherein the myocardial infarct risk information is generated, wherein the myocardial infarct risk is generated by applying (APP-3) the trained risk determination function to the image based raw risk features, the first confidence value, the supplementary raw risk features and the second confidence value, wherein the confidence values can be used by the trained risk determination function to weight the influence of features on the myocardial infarction risk information,
- providing (PRO-1) the determined myocardial infarct risk information.

2. Computer-implemented method according to claim 1, wherein the myocardial infarct risk information comprises a probability and/or probability confidence value for a myocardial infarct, hospital readmission due to a myocardial infarct and/or death due to a myocardial infarct.

3. Computer-implemented method according to one of the previous claims, wherein the medical imaging data comprise coronary angiography data, wherein by applying (APP-1) the trained image analysis function to the medical imaging data as image based raw risk a coronary stenosis, a level of an artery narrowing, artery abnormality, wall irregularities, tortuosity and/or myocardial bridging is determined, localized, segmented and/or labeled.

4. Computer-implemented method according to one of the previous claims, wherein the clinical, demographic, personal and/ or survey data of the supplementary data comprise blood values, genetics, medical condition, smoking status, potential risk factors and/or a preprocessed scoring result for a risk factor.

5. Computer-implemented method according to one of the previous claims, further comprising:

- receiving update data for the patient, wherein the update data are provided by a wearable device or periodic questionnaires after applying (APP-3) the trained risk determination function to the image based raw risk features and to the supplementary raw risk features, wherein the update data comprise monitoring data of physical activities, fitness or vital parameters and/or information on medical treatment compliance, wherein the trained risk determination function is applied to the image based raw risk features, the supplementary raw risk features and the update data, wherein an updated myocardial infarct risk information is determined.

6. A computer-implemented method for providing a trained risk determination function, comprising:

- receiving (REC-3) image based raw risk features derived from medical imaging data of at least a section of the heart of a patient, wherein the received image based raw risk features comprise at least one first confidence value,
- receiving (REC-4) supplementary raw risk features derived from supplementary data comprising clinical, demographic, personal and/or survey data of the patient, wherein the received supplementary raw risk features comprise at least one second confidence value,
- receiving (REC-5) input myocardial infarct risk information of the patient,
- applying (APP-4) a trained risk determination function to the image based raw risk features and to the supplementary raw risk features, wherein output myocardial infarct risk information is generated,
- training (TRA-1) the trained risk determination function based on a comparison of the input myocardial infarct risk information and the output myocardial infarct risk information,
- providing (PRO-2) the trained risk determination function.

7. Computer-implemented method according to claim 6, further comprising:

- receiving (REC-6) image training data, wherein the image training data comprise medical imaging data,
- receiving (REC-8) supplementary training data,
- receiving (REC-7) input image based raw risk features for the image training data,
- receiving (REC-9) input supplementary raw risk features for the supplementary training data,
- applying (APP-5) a trained image analysis function to the image training data, wherein output image based raw risk features are generated,
- applying (APP-6) a trained data analysis function to the supplementary training data, wherein output supplementary raw risk features are generated,

- training (TRA-2) the trained image analysis function based on a comparison of the input image based raw risk features and the output image based raw risk features,
- training (TRA-3) the trained data analysis function based on a comparison of the input supplementary raw risk features and the output supplementary raw risk features,
- providing (PRO-3) the trained image analysis function,
- providing (PRO-3) the trained data analysis function.

8. Computer-implemented method according to claim 7, further comprising:

- providing (PRO-3) the output image based raw risk features as the received image based raw risk features,
- providing (PRO-3) the output supplementary raw risk features as the received supplementary raw risk features.

9. Computer-implemented method according to one of the claims 6 to 8, wherein the input myocardial infarct risk information, input image based raw risk features and/or input supplementary raw risk features are generated based on mortality data of former patients due to a myocardial infarct, hospitalization of former patients due to a myocardial infarct, interviews with former myocardial infarct patients or relatives of the patients.

10. Computer-implemented method according to one of the claims 6 to 9, wherein the input myocardial infarct risk information, input image based raw risk features and/or input supplementary raw risk features are generated based on programmatic creation, wherein labeling functions based on weak supervision strategies or domain heuristics are used to label subsets of data.

11. The method according to one of the claims 1 to 5, wherein the trained risk determination function was provided by the method according to one of the claims 6 to 10.

12. A risk determination system, comprising:

- a first interface (SYS-IF), configured for receiving medical imaging data and supplementary data,
- a second interface (SYS-IF), configured for providing a myocardial infarct risk information,
- a computation unit (SYS-CU), configured for applying a trained risk determination function to the input data and for executing and/or applying the method for determining the myocardial infarct risk information according to one of the claims 1 to 5,

wherein the output data is generated.

13. A computer program product with a computer program, which is able to be loaded directly into a memory of a risk determination system according to claim 12, with program sections for carrying out all steps of the method according to one of the claims 1 to 5 when the program sections are executed by the risk determination system.

14. A computer-readable memory medium, on which program sections able to be read and executed by a risk determination system according to claim 12 are stored, for carrying out all steps of the method according to one of the claims 1 to 5 when the program sections are executed by the risk determination system.

**Patentansprüche**

1. Computerimplementiertes Verfahren zur Bestimmung von Myokardinfarkt-Risikoinformationen für einen Patienten basierend auf medizinischen Bildgebungsdaten und auf ergänzenden Daten, wobei das Verfahren umfasst:

- Empfangen (REC-1) medizinischer Bildgebungsdaten von zumindest einem Abschnitt des Herzens des Patienten,
- Empfangen (REC-2) ergänzender Daten, umfassend klinische, demographische, persönliche und/oder Erhebungsdaten des Patienten,
- Anwenden (APP-1) einer trainierten Bildanalysefunktion auf die medizinischen Bildgebungsdaten, wobei bildbasierte rohe Risikomerkmale generiert werden, wobei Anwenden (APP-1) der trainierten Bildanalysefunktion auf die medizinischen Bildgebungsdaten einen ersten Konfidenzwert für die generierten bildbasierten rohen Risikomerkmale generiert,
- Anwenden (APP-2) einer trainierten Datenanalysefunktion auf die ergänzenden Daten, wodurch ergänzende rohe Risikomerkmale generiert werden, wobei Anwenden (APP-2) der trainierten Datenanalysefunktion auf die ergänzenden Daten einen zweiten Konfidenzwert für die generierten ergänzenden rohen Risikomerkmale generiert,
- Anwenden (APP-3) einer trainierten Risikobestimmungsfunktion auf die bildbasierten rohen Risikomerkmale und auf die ergänzenden rohen Risikomerkmale, wodurch die Myokardinfarkt-Risikoinformationen generiert werden, wobei das Myokardinfarktrisiko durch Anwenden (APP-3) der trainierten Risikobestimmungsfunktion auf die bildbasierten rohen Risikomerkmale, den ersten Konfidenzwert, die ergänzenden rohen Risikomerkmale und den zweiten Konfidenzwert generiert wird, wobei die Konfidenzwerte von der trainierten Risikobestimmungsfunktion verwendet werden können, um den Einfluss von Merkmalen auf die Myokardinfarkt-Risikoinformationen zu gewichten,

- Bereitstellen (PRO-1) der bestimmten Myokardinfarkt-Risikoinformationen.

**2.** Computerimplementiertes Verfahren nach Anspruch 1, wobei die Myokardinfarkt-Risikoinformationen einen Wahrscheinlichkeitswert und/oder Wahrscheinlichkeits-Konfidenzwert für einen Myokardinfarkt, erneute Aufnahme im Krankenhaus aufgrund eines Myokardinfarkts und/oder Tod aufgrund eines Myokardinfarkts umfassen.

**3.** Computerimplementiertes Verfahren nach einem der vorhergehenden Ansprüche, wobei die medizinischen Bildgebungsdaten Koronarangiographiedaten umfassen, wobei durch Anwenden (APP-1) der trainierten Bildanalysefunktion auf die medizinischen Bildgebungsdaten als bildbasiertes rohes Risiko eine Koronarstenose, ein Niveau einer Arterienverengung, Arterienanomalität, Wandunregelmäßigkeiten, Tortuosität und/oder Myokardbrücke bestimmt, lokalisiert, segmentiert und/oder gekennzeichnet wird/werden.

**4.** Computerimplementiertes Verfahren nach einem der vorhergehenden Ansprüche, wobei die klinischen, demographischen, persönlichen und/oder Erhebungsdaten der ergänzenden Daten Blutwerte, Genetik, Gesundheitszustand, Raucherstatus, potenzielle Risikofaktoren und/oder ein vorverarbeitetes Scoring-Ergebnis für einen Risikofaktor umfassen.

**5.** Computerimplementiertes Verfahren nach einem der vorhergehenden Ansprüche, ferner umfassend:

- Empfangen von Aktualisierungsdaten für den Patienten, wobei die Aktualisierungsdaten durch ein tragbares Gerät oder regelmäßige Fragebögen nach Anwenden (APP-3) der trainierten Risikobestimmungsfunktion auf die bildbasierten rohen Risikomerkmale und auf die ergänzenden rohen Risikomerkmale bereitgestellt werden, wobei die Aktualisierungsdaten Überwachungsdaten über körperliche Aktivitäten, Fitness- oder Vitalparameter und/oder Informationen zur Compliance bei medizinischen Behandlungen umfassen, wobei die trainierte Risikobestimmungsfunktion auf die bildbasierten rohen Risikomerkmale, die ergänzenden rohen Risikomerkmale und die Aktualisierungsdaten angewendet wird, wodurch aktualisierte Myokardinfarkt-Risikoinformationen bestimmt werden.

**6.** Computerimplementiertes Verfahren zum Bereitstellen einer trainierten Risikobestimmungsfunktion, umfassend:

- Empfangen (REC-3) bildbasierter roher Risikomerkale, die aus medizinischen Bildgebungsdaten zumindest eines Herzabschnitts eines Patienten abgeleitet sind, wobei die empfangenen bildbasierten rohen Risikomerkmale zumindest einen ersten Konfidenzwert umfassen,
- Empfangen (REC-4) ergänzender roher Risikomerkmale, die aus ergänzenden Daten abgeleitet sind, umfassend klinische, demographische, persönliche und/oder Erhebungsdaten des Patienten, wobei die empfangenen ergänzenden rohen Risikomerkmale zumindest einen zweiten Konfidenzwert umfassen,
- Empfangen (REC-5) von Myokardinfarkt-Risikoinformationen des Patienten der Eingabe,
- Anwenden (APP-4) einer trainierten Risikobestimmungsfunktion auf die bildbasierten rohen Risikomerkmale und die ergänzenden rohen Risikomerkmale, wodurch Myokardinfarkt-Risikoinformationen der Ausgabe generiert werden,
- Trainieren (TRA-1) der trainierten Risikobestimmungsfunktion basierend auf einem Vergleich der Myokardinfarkt-Risikoinformationen der Eingabe und der Myokardinfarkt-Risikoinformationen der Ausgabe,
- Bereitstellen (PRO-2) der trainierten Risikobestimmungsfunktion.

**7.** Computerimplementiertes Verfahren nach Anspruch 6, ferner umfassend:

- Empfangen (REC-6) von Bildtrainingsdaten, wobei die Bildtrainingsdaten medizinische Bildgebungsdaten umfassen,
- Empfangen (REC-8) von ergänzenden Trainingsdaten,
- Empfangen (REC-7) von bildbasierten rohen Risikomerkmalen der Eingabe für die Bildtrainungsdaten,
- Empfangen (REC-9) ergänzender roher Risikomerkmale der Eingabe für die ergänzenden Trainingsdaten,
- Anwenden (APP-5) einer trainierten Bildanalysefunktion auf die Bildtrainingsdaten, wobei bildbasierte rohe Risikomerkmale der Ausgabe generiert werden,
- Anwenden (APP-6) einer trainierten Datenanalysefunktion auf die ergänzenden Trainungsdaten, wobei ergänzende rohe Risikomerkmale der Ausgabe generiert werden,
- Trainieren (TRA-2) der trainierten Bildanalysefunktion basierend auf einem Vergleich der bildbasierenden rohen Risikomerkmale der Eingabe und der bildbasierenden rohen Risikomerkmale der Ausgabe,
- Trainieren (TRA-3) der trainierten Datenanalysefunktion basierend auf einem Vergleich der ergänzenden rohen Risikomerkmale der Eingabe und der ergänzenden rohen Risikomerkmale der Ausgabe,
- Bereitstellen (PRO-3) der trainierten Bildanalysefunktion,
- Bereitstellen (PRO-3) der trainierten Datenanalysefunktion.

**8.** Computerimplementiertes Verfahren nach Anspruch 7, ferner umfassend:

- Bereitstellen (PRO-3) der bildbasierenden rohen Risikomerkmale der Ausgabe als die empfangenen bildbasierten rohen Risikomerkmale,
- Bereitstellen (PRO-3) der ergänzenden rohen Risikomerkmale der Ausgabe als die empfangenen ergänzenden rohen Risikomerkmale.

**9.** Computerimplementiertes Verfahren nach einem der Ansprüche 6 bis 8, wobei die Myokardinfarkt-Risikoinformationen der Eingabe, bildbasierten rohen Risikomerkmale der Eingabe und/oder ergänzenden rohen Risikomerkmale der Eingabe basierend auf Mortalitätsdaten ehemaliger Patienten aufgrund eines Myokardinfarkts, Hospitalisierung ehemaliger Patienten aufgrund eines Myokardinfarkts, Interviews mit ehemaligen Myokardinfarktpatienten oder Verwandten der Patienten generiert werden.

**10.** Computerimplementiertes Verfahren nach einem der Ansprüche 6 bis 9, bei dem die Myokardinfarkt-Risikoinformationen der Eingabe, bildbasierten rohen Risikomerkmale der Eingabe und/oder ergänzenden rohen Risikomerkmale der Eingabe basierend auf programmatischer Erstellung generiert werden, wobei Kennzeichnungsfunktionen auf Basis schwacher Überwachungsstrategien oder Domänenheuristiken verwendet werden, um Teilmengen von Daten zu kennzeichnen.

**11.** Verfahren nach einem der Ansprüche 1 bis 5, wobei die trainierte Risikobestimmungsfunktion durch das Verfahren nach einem der Ansprüche 6 bis 10 bereitgestellt wurde.

**12.** Risikobestimmungssystem, umfassend:

- eine erste Schnittstelle (SYS-IF), die zum Empfangen medizinischer Bildgebungsdaten und ergänzender Daten ausgelegt ist,
- eine zweite Schnittstelle (SYS-IF), die zum Bereitstellen von Myokardinfarkt-Risikoinformationen ausgelegt ist,
- eine Recheneinheit (SYS-CU), die zum Anwenden einer trainierten Risikobestimmungsfunktion auf die Eingabedaten und zum Ausführen und/oder Anwenden des Verfahrens zum Bestimmen der Myokardinfarkt-Risikoinformationen nach einem der Ansprüche 1 bis 5 ausgelegt ist,

wobei die Ausgabedaten generiert werden.

**13.** Computerprogrammprodukt mit einem Computerprogramm, das direkt in einen Speicher eines Risikobestimmungssystems nach Anspruch 12 geladen werden kann, mit Programmabschnitten zum Durchführen aller Schritte des Verfahrens nach einem der Ansprüche 1 bis 5, wenn die Programmabschnitte durch das Risikobestimmungssystem ausgeführt werden.

**14.** Computerlesbares Speichermedium, auf dem Programmabschnitte gespeichert sind, die von einem Risikobestimmungssystem nach Anspruch 12 gelesen und ausgeführt werden können, zum Durchführen aller Schritte des Verfahrens nach einem der Ansprüche 1 bis 5, wenn die Programmabschnitte durch das Risikobestimmungssystem ausgeführt werden.

**Revendications**

**1.** Un procédé mis en œuvre par ordinateur de détermination d'une information de danger d'infarctus du myocarde pour un patient sur la base de données d'imagerie médicale et de données supplémentaires, le procédé comprenant :

- recevoir (REC-1) des données d'imagerie médicale d'au moins une partie du cœur du patient,
- recevoir (REC-2) des données supplémentaires comprenant des données cliniques, démographiques, personnelles et/ou générales du patient,
- appliquer (APP-1) une fonction entraînée d'analyse d'image aux données d'imagerie médicale, dans lequel des caractéristiques brutes de danger fondées sur l'image sont créées, dans lequel appliquer (APP-1) la fonction entraînée d'analyse d'image aux données d'imagerie médicale crée une première valeur de confiance de l'image créée sur la base de caractéristiques brutes de danger,

- appliquer (APP-2) une fonction entraînée d'analyse d'image aux données supplémentaires, des caractéristiques brutes supplémentaires de danger étant créées, dans lequel appliquer (APP-2) la fonction entraînée d'analyse d'image aux données supplémentaires crée une deuxième valeur de confiance des caractéristiques brutes supplémentaires créées de danger,

- appliquer (APP-3) une fonction entraînée de détermination de danger aux caractéristiques brutes de danger fondées sur l'image et aux caractéristiques brutes supplémentaires de danger, l'information de danger d'infarctus du myocarde étant créée, dans lequel le danger d'infarctus du myocarde est créé en appliquant (APP-3) la fonction entraînée de détermination du danger aux caractéristiques brutes de danger, fondées sur l'image à la première valeur de confiance, aux caractéristiques brutes supplémentaires de danger et à la deuxième valeur de confiance, dans lequel les valeurs de confiance peuvent être utilisées par la fonction entraînée de détermination du danger pour pondérer l'influence de caractéristiques sur l'information de danger d'infarctus du myocarde,

- donner (PRO-1) l'information déterminée de danger d'infarctus du myocarde.

2. Procédé mis en œuvre par ordinateur suivant la revendication 1, dans lequel l'information de danger d'infarctus du myocarde comprend une probabilité et/ou une valeur de confiance de probabilité d'un infarctus du myocarde, une réadmission en hôpital dûe à un infarctus du myocarde et/ou une mort due à un infarctus du myocarde.

3. Procédé mis en œuvre par ordinateur suivant l'une des revendications précédentes, dans lequel les données d'imagerie médicale comprennent des données d'angiographie coronaire, dans lequel en appliquant (APP-1), la fonction entraînée d'analyse d'image aux données d'imagerie médicale comme danger brut reposant sur l'image, il est déterminé, localisé, segmenté et/ou marqué une sténose coronaire, un niveau de rétrécissement d'artère, une anormalité d'artère, des irrégularités de paroi, une tortuosité et/ou un pontage myocardial.

4. Procédé mis en œuvre par ordinateur suivant l'une des revendications précédentes, dans lequel les données cliniques, démographiques, personnelles et/ou générales des données supplémentaires comprennent des valeurs sanguines, de la génétique, un état médical, un état de fumeur, des facteurs potentiels de danger et/ou un résultat de score prétraité pour un facteur de danger.

5. Procédé mis en œuvre par ordinateur suivant l'une des revendications précédentes, comprenant en outre :

- recevoir des données mises à jour du patient, dans lequel les données mises à jour sont données par un dispositif portable ou par des questionnaires périodiques après avoir appliqué (APP-3), la fonction entraînée de détermination du danger aux caractéristiques brutes de danger, fondées sur l'image et aux caractéristiques brutes supplémentaires de danger, dans lequel les données mises à jour comprennent contrôler des données d'activités physiques, des paramètres de bonne forme ou vitaux et/ou de l'information sur l'acceptation du traitement médical, dans lequel la fonction entraînée de détermination du danger est appliquée aux caractéristiques brutes de danger, fondées sur l'image, aux caractéristiques brutes supplémentaires de danger et aux données mises à jour, une information mise à jour de danger d'infarctus du myocarde étant déterminée.

6. Un procédé mis en œuvre par ordinateur d'obtention d'une fonction entraînée de détermination de danger, comprenant :

- recevoir (REC-3) des caractéristiques brutes de danger, fondées sur l'image déduites de données d'imagerie médicale d'au moins une partie du cœur d'un patient, dans lequel les caractéristiques brutes de danger fondées sur l'image comprennent au moins une première valeur de confiance,

- recevoir (REC-4) des caractéristiques brutes supplémentaires de danger, déduites de données supplémentaires comprenant des données cliniques, démographiques, personnelles et/ou générales du patient, dans lequel les caractéristiques brutes supplémentaires reçues de danger comprennent au moins une deuxième valeur de confiance,

- recevoir (REC-5) de l'information d'entrée de danger d'infarctus du myocarde du patient,

- appliquer (APP-4) une fonction entraînée de détermination de danger aux caractéristiques brutes de danger, fondées sur l'image et aux caractéristiques brutes supplémentaires de danger, une information de sortie d'infarctus du myocarde étant créée,

- entraîner (TRA-1) la fonction entraînée de détermination du danger sur la base d'une comparaison entre l'information d'entrée du danger d'infarctus du myocarde et l'information de sortie du danger d'infarctus du myocarde,

- donner (PRO-2) la fonction entraînée de détermination de danger.

7. Procédé mis en œuvre par ordinateur suivant la revendication 6, comprenant en outre :

- recevoir (REC-6) des données d'entraînement d'image, dans lequel les données d'entraînement d'image comprennent des données d'imagerie médicale,
- recevoir (REC-8) des données supplémentaires d'entraînement,
- recevoir (REC-7) des caractéristiques brutes de danger d'entrée fondées sur l'image pour les données d'entraînement d'image,
- recevoir (REC-9) des caractéristiques brutes de danger supplémentaires d'entrée pour les données d'entraînement supplémentaires,
- appliquer (APP-5) une fonction entraînée d'analyse d'image aux données d'entraînement d'image, des caractéristiques brutes de danger de sortie fondées sur l'image étant créées,
- appliquer (APP-6) une fonction entraînée d'analyse de données aux données d'entraînement supplémentaires, des caractéristiques brutes supplémentaires de danger de sortie étant créées,
- entraîner (TRA-2) la fonction entraînée d'analyse d'image sur la base d'une comparaison entre les caractéristiques brutes de danger d'entrée fondées sur l'image et les caractéristiques brutes de danger de sortie fondées sur l'image,
- entraîner (TRA-3) la fonction entraînée d'analyse de données sur la base d'une comparaison entre les caractéristiques brutes de danger supplémentaires d'entrée et les caractéristiques brutes de danger supplémentaires de sortie,
- donner (PRO-3) la fonction entraînée d'analyse d'image,
- donner (PRO-3) la fonction entraînée d'analyse de données.

8. Procédé mis en œuvre par ordinateur suivant la revendication 7, comprenant en outre :

- donner (PRO-3) les caractéristiques brutes de danger de sortie fondées sur l'image comme caractéristiques brutes de danger reçues fondées sur l'image,
- donner (PRO-3) les caractéristiques brutes de danger supplémentaires de sortie comme caractéristiques brutes de danger supplémentaires reçues.

9. Procédé mis en œuvre par ordinateur suivant l'une des revendications 6 à 8, dans lequel l'information d'entrée de danger d'infarctus du myocarde, les caractéristiques brutes de danger d'entrée fondées sur l'image et/ou les caractéristiques brutes de danger supplémentaires d'entrée sont créées sur la base de données de mortalité de patients antérieurs due à un infarctus du myocarde, une hospitalisation de patients antérieurs dues à un infarctus du myocarde, des interviews avec des patients antérieurs ayant eu un infarctus du myocarde ou avec des relations des patients.

10. Procédé mis en œuvre par ordinateur suivant l'une des revendications 6 à 9, dans lequel l'information de danger d'infarctus du myocarde, des caractéristiques brutes de danger d'entrée fondées sur l'image et/ou des caractéristiques brutes de danger supplémentaires d'entrée sont créées sur la base d'une création programmatique, dans lequel des fonctions de marquage fondées sur des stratégies de supervision faibles ou des heuristiques de domaine sont utilisées pour marquer des sous-jeux de données.

11. Le procédé suivant l'une des revendications 1 à 5, dans lequel la fonction entraînée de détermination de danger est donnée par le procédé suivant l'une des revendications 6 à 10.

12. Un système de détermination de danger, comprenant :

- une première interface (SYS-IF), configurée pour recevoir des données d'imagerie médicale et des données supplémentaires,
- une deuxième interface (SYS-IF), configurée pour donner une information de danger d'infarctus du myocarde,
- une unité (SYS-CU) informatique, configurée pour appliquer une fonction entraînée de détermination de danger aux données d'entrée pour exécuter et/ou appliquer le procédé de détermination de l'information de danger d'infarctus du myocarde suivant l'une des revendications 1 à 5,

la donnée de sortie étant créée.

13. Un produit de programme d'ordinateur ayant un programme d'ordinateur, qui peut être chargé directement dans la mémoire d'un système de détermination de danger suivant la revendication 12, ayant des parties de programme pour effectuer tous les stades du procédé suivant l'une des revendications 1 à 5, lorsque les parties de programme sont exécutées par le système de détermination de danger.

14. Un support de mémoire déchiffrable par ordinateur, sur lequel des parties de programme, pouvant être lues et exécutées par un système de détermination de danger suivant la revendication 12, sont mises en mémoire, afin d'effectuer tous les stades du procédé suivant l'une des revendications 1 à 5, lorsque les parties de programme sont exécutées par le système de détermination de danger.

## FIG 1

REC-1 — [ ]

REC-2 — [ ]

APP-1 — [ ]

APP-2 — [ ]

APP-3 — [ ]

PRO-1 — [ ]

## FIG 2

REC-3   REC-4   REC-5

[ ]   [ ]   [ ]

APP-4 — [ ]

TRA-1 — [ ]

PRO-2 — [ ]

# FIG 3

REC-6

REC-8

APP-5

REC-7

APP-6

REC-9

TRA-3

TRA-2

PRO-3

REC-3, REC-4

APP-4

REC-5

TRA-1

PRO-2

# FIG 4

SYS-IF

SYS-CU

SYS

SYS-M4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3404667 A1
- US 2021251577 A1

- US 2018310888 A1

**Non-patent literature cited in the description**

- SYNTAX score based on coronary computed tomography angiography may have a prognostic value in patients with complex coronary artery disease: An observational study from a retrospective cohort.. *Medicine*, 2017, vol. 96 (37), e7999